# EUROPEAN PATENT APPLICATION

(11) **EP 1 623 688 A1**
(43) Date of publication of application: **08.02.2006**
(21) Application number: 05380163.5
(22) Date of filing: 20.07.2005
(51) Int. Cl.: A61F 5/44, A61M 39/22, B65D 47/26, F16K 5/04

(54) **Tap for clinical use**

(30) Priority: 06.08.2004 ES 200401923 U
(71) Applicant: TECNOCLINIC, S.A., 08208 Sabadell (ES)
(72) Inventor: Caval Marques, Albert, 08208 Sabadell (ES)
(74) Representative: Marques Alos, Fernando

(57) **Abstract**

"TAP FOR CLINICAL USE" of the type specially applicable for urine bags, characterised essentially by being constituted basically of an inner cylinder and an outer cylinder, the inner cylinder having a channel passing through it in such a way that the entry at one end of the cylinder and the exit at the other end.

## Description

The subject of this invention, as expressed in the heading of this descriptive memorandum, consists of a tap for clinical use, of the type used to open and close the passage at the exit of a bag for collecting urine.

There exist currently various models of taps for urine bags, operated by a lever or wing nut, which are based on the principle of a quarter turn (that is, a turn of approximately 90 degrees of angle) so that in position 0 degrees, the tap remains closed while in position 90 degrees, the tap is open.

When hospitals, clinics or other health centres connect a patient to a bag for collecting urine provided with a tap for emptying, it often happens, particularly at night and in order to increase the collection capacity of the system, that a second bag is added, connected in tandem to the tap of the first. Consequently, during the night, the normal position of a standard tap for a urine bag is generally open, that is, at 90 degrees, as the drainage of urine from the patient must be constant.

This has the substantial disadvantage that the 90 degrees position (open) means that the lever or wing nut is in a position at right-angles with respect to the tube of the bag, making it very easy for the patient or any other person to knock it accidentally and close it (position 0 degrees). A situation such as that described would be especially dangerous should the patient be asleep or distracted for any other reason, as the urine would be accumulating indefinitely in the bag until it overflowed with the consequent risk of infections (and other damage) which this could cause to the patient.

In order to eliminate this and other drawbacks of a technical and health nature, a new tap for the urine bag has been created with a lever designed in such a way that in the "closed" position the lever is at 0 degrees with respect to the bag tube (that is, parallel to it), while in the "open" position the lever is at approximately 180 degrees from its initial position, that is to say, a half turn (and, therefore, again parallel to the bag tube), so that it is virtually impossible to close the tap accidentally.

The new tap is formed basically by two pieces: an inner cylinder and an outer cylinder.

The inner cylinder has a channel passing through it in such a way that the entry to it is on one side and the exit on the other side. Thus, this channel can adopt two configurations, that of a perforation passing diagonally and of constant section or that of a perforation of progressively decreasing section with one vertical side and another oblique, so that the upper mouth of the channel presents a larger area than the lower mouth. Particularly this last solution is effectively of greater application, in the facility it gives in the phase of moulding the piece, as against the option of the diagonal perforation.

This inner cylinder has attached to its outer part the action lever and a tab which acts as a stop, placed in diametrically opposed positions. It also has a circular outer crest which facilitates its fitting into the outer cylinder.

The outer cylinder, inside which the inner cylinder is lodged, has at its two ends, two channels, one in the upper part and the other in the lower part, for the entry and exit respectively, of the liquid.

It also has a flange which acts as the final stop in the turn of the inner cylinder, arranged so that it comes up against the flange of the inner cylinder when the have turn is made in passing from the "normally open" position to "closed".

These two channels in the outer cylinder are positioned strategically to coincide with the entry and exit of the diagonal channel of the inner cylinder.

The outer cylinder also has a circular guide, inside it, into which the outer circular crest of the inner cylinder fits.

The functioning of the novel tap is very simple and is described hereunder.

When the tap is in the "open" position, the lever of the inner cylinder is downwards; in this situation, the upper and lower channels of the outer cylinder and the diagonal channel of the inner cylinder are matched so that the liquid flows through the tap without problems.

When the inner cylinder is turned 180° its diagonal channel is out of phase with the channels of the outer cylinder and stops the flow of liquid.

In both positions, the tap lever is aligned with the drainage tubes, which avoids to a great degree the accidental movements of closure.

### DESCRIPTION OF THE DRAWINGS

In order to facilitate understanding of which has been explained above, a sheet of drawings is attached to this descriptive memorandum, forming an integral part of it, showing in a simplified and schematic way an example of the novel tap, for purposes merely of illustration and not limiting the practical possibilities of execution.

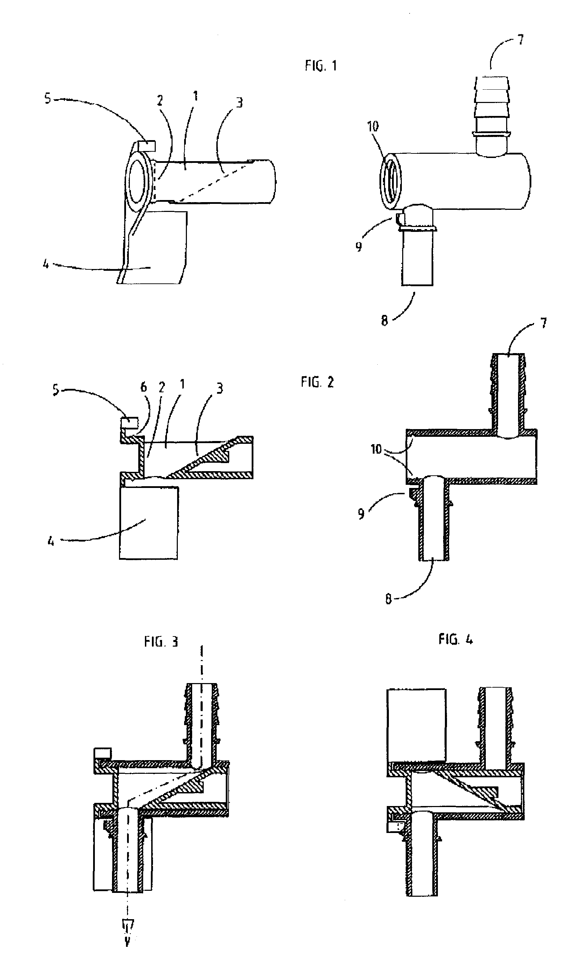

In these drawings, fig. 1 shows the parts of the tap in the "open" position.

Fig. 2 shows a view in lateral section of the two parts of the tap, taken apart.

Fig. 3 shows a view in lateral section of the tap in the open position.

Fig. 4 shows a view in lateral section of the tap in the closed position.

### DESCRIPTION OF A PRACTICAL CASE

The novel tap which it is sought to patent consists of an inner cylinder with a vertical channel through it of progressively decreasing section (1). The front section of this orifice shows a vertical limit on one side (2) and oblique on the other (3); made like this, the upper mouth of the channel has greater width than the lower mouth.

The inner cylinder has attached to its outer part an action lever (4) and a tab or flange as a stop (5), both elements placed in diametrically opposed positions.

The inner cylinder is inserted into an outer cylinder, which has at its two distal ends, two channels, one in the upper part (7) and the other in the lower part (8), corresponding to the entry and exit of the liquid.

The lower channel (8), has attached to its outer part a stop flange (9) which comes up against the stop flange (5) of the inner cylinder when this is given a half turn, in passing from the "open" position to "closed".

When the tap is open these two channels in the outer cylinder (7 and 8), communicate with the channel of the inner cylinder (1) allowing the urine to pass.

The outer cylinder has a circular guide or groove inside it (10), into which the circular outer crest or ridge (6) of the inner cylinder fits, ensuring its safe coupling.

The functioning of the novel tap is as follows:

When the tap is in the "open" position, the lever of the inner cylinder is downwards; in this situation, the upper channel of the outer cylinder (7) is opposite the wide mouth of the channel through the inner cylinder (1) and the liquid flows with no problems.

On giving the lever a half turn, the stop of the inner cylinder (5) meets the stop of the outer cylinder (9) and the turning movement ends. In this position of the inner cylinder, its channel (1) is with the narrow mouth upwards and the wide mouth downwards.

When this happens, the upper channel of the outer cylinder (7) is not opposite the narrow mouth of the channel of the inner cylinder (1) and, then, the liquid cannot flow.

The materials used in the manufacture of the different elements composing this invention are independent of its subject, as are the shapes, dimensions and accessories which may be present, being able to be replaced by others technically equivalent, provided that they do not affect the essence of it nor are outside the ambit defined in the claims section.

Having established the concept expressed, the note of claims is set out below, summarising the novelties which it is wished to claim:

## Claims

1. "TAP FOR CLINICAL USE" of the type specially applicable for urine bags, **characterised** essentially by being constituted basically of an inner cylinder and an outer cylinder, the inner cylinder having a channel passing through it in such a way that the entry at one end of the cylinder and the exit at the other end.

2. "TAP FOR CLINICAL USE" according to the first claim, **characterised** because the channel of the inner cylinder is formed as a perforation passing diagonally, of constant section.

3. "TAP FOR CLINICAL USE" according to the first claim, **characterised** because the channel of the inner cylinder is constituted by a perforation of progressively decreasing section, with one side vertical and the other oblique, so that the upper mouth of the channel presents a larger area than the lower mouth.

4. "TAP FOR CLINICAL USE" according to any of the above claims, **characterised** because the inner cylinder has attached on its outer part an action lever and a flange acting as a stop, placed in diametrically opposed positions.

5. "TAP FOR CLINICAL USE" according to any of the above claims, **characterised** because the inner cylinder has a circular exterior ridge which fits into a guide or circular groove present in the lower part of the outer cylinder.

6. "TAP FOR CLINICAL USE" according to any of the above claims, **characterised** because the outer cylinder, inside which the inner cylinder lodges, has at its two ends two channels perpendicular to it, one above and the other below, for the entry and exit of urine in the tap, and a flange which acts as a stop in the turn of the inner cylinder, placed so that the flange of the inner cylinder meets it when making the movement to pass from the "open" to "closed" position.

7. "TAP FOR CLINICAL USE" according to any of the above claims, **characterised** because the channels of the outer cylinder are placed strategically to coincide with the entry and exit of the channel through the inner cylinder when the tap is in the open position, the lever of the inner cylinder being lined up with the channels of the outer cylinder, while on turning the inner cylinder by 180°, to the closed position, the channel through it is out of phase with the channels of the outer cylinder, while the lever of the inner cylinder is again lined up with the channels of the outer cylinder.
